# EUROPÄISCHE PATENTSCHRIFT

(11) **EP 3 011 006 B1**
(45) Veröffentlichungstag und Bekanntmachung des Hinweises auf die Patenterteilung: **31.05.2017**
(21) Anmeldenummer: 13805300.4
(22) Anmeldetag: 28.11.2013
(51) Int. Cl.: C12M 1/00, B29D 23/00, C12M 1/12, B29C 47/00, B29C 47/06

(54) **ROHRLEITUNG ZUM EINSATZ IN EINEM PHOTOBIOREAKTOR**
TUBING FOR USE IN A PHOTOBIOREACTOR
CANALISATION DESTINÉE À ÊTRE UTILISÉE DANS UN PHOTOBIORÉACTEUR

(30) Priorität: 20.06.2013 DE 102013106478
(43) Veröffentlichungstag der Anmeldung: 27.04.2016
(73) Patentinhaber: BioTubes GmbH, 59457 Werl (DE)
(72) Erfinder: KAISER, Hans, 59757 Arnsberg (DE); KAISER, Hubertus, 59755 Arnsberg (DE); BARTON, Philip, E-07181 Calvia/Mallorca (ES)
(74) Vertreter: Patentanwälte Dörner & Kötter PartG mbB
(86) Internationale Anmeldenummer: PCT/EP2013/074985
(87) Internationale Veröffentlichungsnummer: WO 2014/202159

(56) Entgegenhaltungen:
- EP-A1- 2 284 218
- WO-A1-02/099031
- GB-A- 997 551
- US-A1- 2010 248 333

## Beschreibung

Die Erfindung betrifft eine Rohrleitung zum Einsatz in einem Photobioreaktor zur Herstellung von Mikroalgenkonzentrat nach dem Oberbegriff des Patentanspruchs 1. Die Erfindung betrifft weiterhin ein Verfahren zur Herstellung eines solchen Rohres nach dem Patentanspruch 13.

Eine Anlage zur Herstellung von Mikroalgenkonzentrat ist beispielsweise in der DE 103 15 750 A1 beschrieben. Diese Anlage besteht aus einem Photobioreaktor zur Beschleunigung des Wachstums und der Vermehrung der Mikroalgen in einer Suspension mit einer Gruppe von Reaktorsegmenten, die transparente, parallel zueinander ausgerichtete Rohre besitzen sowie aus Behältern zur Anreicherung der Suspension mit Kohlendioxid und anderen Nährstoffen, aus Mitteln zur Abscheidung von Sauerstoff aus der Suspension sowie einer Erntevorrichtung mit Mitteln zur Abtrennung eines Mikroalgenkonzentrates. Dabei sind der Photobioreaktor, der Mischbehälter und die Mittel zum Abscheiden von Sauerstoff in einen Kreislauf eingebunden, der eine Pumpe aufweist. Die Rohre sind in den Reaktorsegmenten parallel zueinander und senkrecht ausgerichtet und in üblicher Weise dem einfallenden Tageslicht, einem äußeren Kunstlicht oder dem Licht von Lichtleitern mit streuendem Lichteffekt ausgesetzt. Nachteilig bei den zum Einsatz kommenden Rohren ist, dass die für die Photosynthese wirksame Oberfläche relativ klein ausgebildet ist, da lediglich der Rohraußenmantel als Lichteinfall- und -austrittsfläche zur Verfügung steht. In der EP 2 036 977 A1 wird hierzu vorgeschlagen, Durchflusstürme einzusetzen, welche durch zwei konzentrisch angeordnete, transluzente Rohre gebildet sind, mit einem Durchmesser von 0,8 bis 1,5 m und einer Höhe von 3 bis 5 m. Die Herstellung derartiger Türme stellt sich in der Praxis jedoch problematisch dar. Insbesondere bereitet der häufig in derartigen Anlagen verwendete Werkstoff Glas bei diesen Dimensionen statische Probleme, da die einzelnen Rohre vor dem Hintergrund der zu erwartenden Druckverhältnisse eine erhebliche Wandstärke aufweisen müssten.

In der WO02099031 A1 wird eine Rohrleitung zum Einsatz in einem Photobioreaktor beschrieben. Die Rohrleitung umfasst zwei konzentrisch angeordnete horizontale Rohre unterschiedlichen Durchmessers. Hier will die Erfindung Abhilfe schaffen. Der Erfindung liegt die Aufgabe zugrunde, eine Rohrleitung zum Einsatz in einem Photobioreaktor zur Herstellung von Mikroalgenkonzentrat bereit zu stellen, die den auftretenden Druckverhältnissen standhält und gleichzeitig geringste Teilchen-Migrationsraten in das Mikroalgenkonzentrat aufweist. Gemäß der Erfindung wird diese Aufgabe durch die Merkmale des kennzeichnenden Teils des Patentanspruchs 1 gelöst.

Mit der Erfindung ist eine Rohrleitung zum Einsatz in einem Photobioreaktor zur Herstellung von Mikroalgenkonzentrat geschaffen, die den auftretenden Druckverhältnissen standhält und zudem eine geringe Teilchen-Migrationsrate aufweist. Durch die Herstellung der beiden Rohre aus lichtdurchlässigem Kunststoff, wobei wenigstens das äußere Rohr über seine Länge wenigstens zwei Abschnitte mit unterschiedlicher Wandstärke aufweist, ist eine leichte und zugleich äußerst robuste Struktur geschaffen, die den auftretenden Druckverhältnissen standhält. Aufgrund des mit zunehmender Konzentratsäule abnehmenden Drucks weist das Rohr auch bei über seine Länge verminderter Wandstärke eine ausreichende Festigkeit auf.

In Weiterbildung der Erfindung weist wenigstens ein Abschnitt eine sich linear verjüngende Wandstärke auf. Hierdurch sind Kerbwirkungen vermieden, wodurch die Belastbarkeit des Rohres erhöht ist. Dabei weisen die Rohre bevorzugt über ihre Länge einen konstanten Querschnitt auf.

In Ausgestaltung der Erfindung sind wenigstens das äußere Rohr, bevorzugt beide Rohre, aus Polymethylmethacrylat (PMMA) hergestellt. Dieses Material ist hochtransparent, witterungsstabil, kostengünstig und gut verarbeitbar.

In alternativer Ausgestaltung sind wenigstens das äußere Rohr, bevorzugt beide Rohre aus Polycarbonat hergestellt und auf ihrer dem Kanal zugewandten Seite mit einer lebensmittelkonformen Schicht und auf ihrer dem Kanal abgewandten Seite mit einer UV-Sperrschicht versehen. Durch die Beschichtung der Rohre auf ihrer jeweiligen dem Kanal zugewandten Seite mit einer lebensmittelkonformen Schicht ist eine geringe Migrationsrate gewährleistet. Durch die Beschichtung der Rohre auf ihrer jeweiligen dem Kanal abgewandten Seite mit einer UV-Sperrschicht ist eine Schädigung der Polycarbonat-Struktur durch UV-Licht verhindert, wodurch eine hohe Standzeit erzielt ist.

In Weiterbildung der Erfindung weisen die beiden Rohre eine Lichtdurchlässigkeit von wenigstens 80%, vorzugsweise wenigstens 85% auf. Hierdurch ist ein hoher Lichteintrag ermöglicht, wodurch der Photosynthese-Prozess beschleunigt ist. Hierzu ist die UV-Sperrschicht bevorzugt derart ausgebildet, dass sie für Lichtwellen mit einer Wellenlänge von < 350 nm undurchlässig und mit einer Wellenlänge von 350 nm bis 700 nm durchlässig ist.

In weiterer Ausgestaltung der Erfindung sind die Sperrschichten aus einem Polycarbonatmaterial gebildet, dem ein UV-Adsorber zugesetzt ist. Hierdurch ist ein homogener Auftrag der UV-Sperrschicht auf das Polycarbonat-Rohr ermöglicht.

In weiterer Ausgestaltung der Erfindung sind die lebensmittelkonformen Schichten aus Polyethylenterephthalat (PET) oder Polycarbonat gebildet. Insbesondere durch die Verwendung von lebensmittelkonformen Polycarbonat, das vorzugsweise den Vorgaben der EU-Verordnung Nr. 10/211 genügt, ist eine homogene Beschichtung des Polycarbonat-Rohres ermöglicht. Dabei sind die UV-Sperrschichten und die lebensmittelkonformen Schichten mit dem jeweiligen Rohr vorzugsweise homogen und ohne Haftvermittler gebunden. Hierdurch sind die optischen Eigenschaften des Rohres möglicherweise beeinträchtigende Klebschichten vermieden.

In weiterer Ausgestaltung der Erfindung sind Mittel zur Verringerung der Lichtbrechung angeordnet, die vorzugsweise durch Prismen und/oder Ziehstreifen gebildet sind. Hierdurch ist der Lichteintrag in den mit Mikroalgenkonzentrat durchströmten Kanal optimiert.

Der Erfindung liegt weiterhin die Aufgabe zugrunde, ein Verfahren zur Herstellung eines Rohres für eine Rohrleitung zum Einsatz in einem Photobioreaktor zur Herstellung von Mikroalgenkonzentrat bereitzustellen, das kostengünstig herstellbar ist und den auftretenden Druckverhältnissen standhält.

Gemäß der Erfindung wird diese Aufgabe durch ein Verfahren mit den Merkmalen des Patentanspruchs 13 gelöst. Dadurch, dass die Rohre in einem Endlos- Extrusionsprozess gefertigt werden, wobei zunächst ein Abschnitt mit einer ersten Wandstärke extrudiert wird, welche Wandstärke bei konstantem Rohraußendurchmesser über die Länge eines sich anschließenden zweiten Abschnitts linear reduziert wird, deren endseitig erreichte Wandstärke über einen sich anschließenden dritten Abschnitt konstant gehalten wird, wobei die vorgenannten Abschnitte nachfolgend in immer wieder umgekehrter Reihenfolge extrudiert werden, wodurch hintereinander jeweils zwei symmetrisch zueinander angeordnete Rohre gebildet sind, die in einem nachfolgenden Arbeitsschritt über einen Schneidprozess voneinander getrennt werden, ist eine kostengünstige Herstellung leichter und zugleich belastbarer Rohre mit kurzen Fertigungszeiten ermöglicht. Dabei werden die Rohre bevorzugt aus PMMA oder Polycarbonat hergestellt.

In Weiterbildung der Erfindung werden die Rohre aus Polycarbonat gefertigt, wobei eine UV-Sperrschicht (13, 23), eine Polycarbonat-Strukturschicht (11, 21) und eine Lebensmittelkontaktschicht (12, 22) coextrudiert werden, wobei das coextrudierte Dreischichtrohr (1, 2) nachfolgend über temperierte und gestaffelte Kühlstufen abgekühlt wird. Dadurch, dass die UV-Schutzschicht die Polycarbonat-Strukturschicht und die Lebensmittelkontaktschicht coextrudiert werden, ist eine gleichmäßige, homogene Beschichtung über die gesamte Rohrlänge bewirkt. Dabei wird das coextrudierte Dreischichtrohr nachfolgend über temperierte und gestaffelte Kühlstufen abgekühlt, wodurch eine spannungsarme Abkühlung der so hergestellten Rohre bewirkt ist. Das so hergestellte Rohr weist eine geringe Teilchen-Migrationsrate auf.

Andere Weiterbildungen und Ausgestaltungen der Erfindung sind in den übrigen Unteransprüchen angegeben. Ein Ausführungsbeispiel der Erfindung ist in der Zeichnung dargestellt und wird nachfolgend im Einzelnen beschrieben. Es zeigen:
- Figur 1: die schematische Darstellung einer Rohrleitung zum Einsatz in einem Photobioreaktor zur Herstellung von Mikroalgenkonzentrat im Querschnitt;
- Figur 2: die schematische Darstellung einer Rohrleitung zum Einsatz in einem Photobioreaktor zur Herstellung von Mikroalgenkonzentrat in einer weiteren Ausführungsform im Längsschnitt und
- Figur 3: die schematische Darstellung der variablen Wandstärke von endlos extrudierten Rohren für Rohrleitungen.

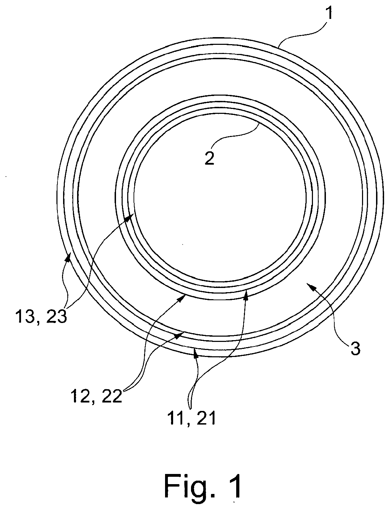

Die als Ausführungsbeispiel gewählte Rohrleitung gemäß Figur 1 ist im Wesentlichen gebildet durch zwei konzentrisch angeordnete Rohre 1, 2 mit unterschiedlichem Durchmesser, zwischen denen ein Kanal 3 zur Durchführung einer Mikroalgensuspension gebildet ist. Das Außenrohr 1 weist im Ausführungsbeispiel eine Wandstärke von 4 mm sowie einen Außendurchmesser von 450 mm auf. An seiner dem Kanal 3 zugewandten Innenmantelfläche ist das Außenrohr 1 mit einer 0,2 mm dicken lebensmittelkonformen Schicht versehen, die aus Polyethylenterephthalat (PET) gebildet ist. Auf seiner dem Kanal 3 abgewandten Außenmantelfläche ist das Rohr 1 mit einer 0,2 mm dicken UV-Sperr-Schicht versehen. Die UV-Sperrschicht 13 ist im Ausführungsbeispiel aus einem mit einem UV-Blocker versehenen Polycarbonat-Werkstoff gebildet.

Das Innenrohr 2 weist im Ausführungsbeispiel eine Wandstärke von 4 mm sowie einen Außendurchmesser von 350 mm auf und ist gleichsam dem Außenrohr 1 an seiner dem Kanal 3 zugewandten Außenmantelfläche mit einer 0,2 mm dicken lebensmittelkonformen Schicht 22 und an seiner dem Kanal 3 abgewandten Innenmantelfläche mit einer 0,2 mm dicken UV-Sperrschicht versehen. Der Kanal 3 weist somit eine Breite von 45,5 mm auf.

Es hat sich gezeigt, dass vorteilhaft der Durchmesser des äußeren Rohres zwischen 300 und 800 mm und der Durchmesser des inneren Rohres zwischen 200 und 700 mm liegen kann. Damit kann die Breite des Kanals 3 für die Mikroalgensuspension ausreichend variiert werden, je nach Konzentration der Algensuspension und Stärke des Lichteinfalls.

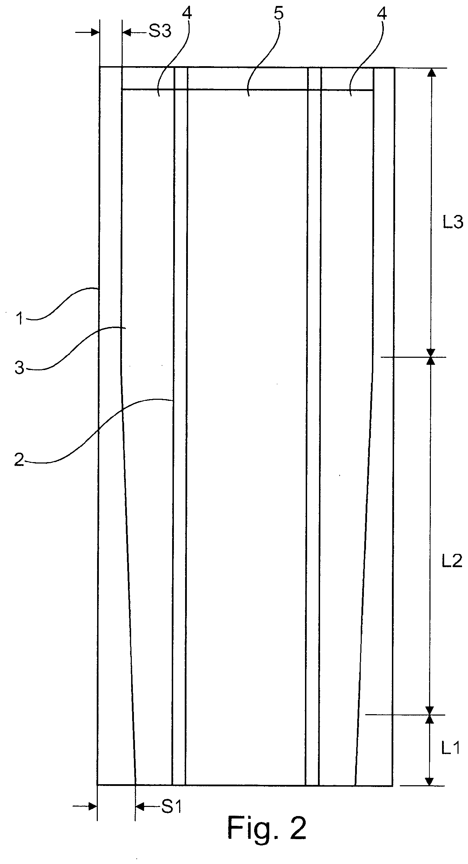

Im Ausführungsbeispiel gemäß Figur 2 sind die Rohre 1, 2 aus PMMA hergestellt. Dabei weist das innere Rohr 2 eine konstante Wandstärke auf, wohingegen das äußere Rohr einen ersten Abschnitt L1 einer Wandstärke von s1 = 6 mm aufweist, an den sich ein zweiter Abschnitt L2 mit linear über seine Länge abnehmender Wandstärke anschließt, der wiederum in einen dritten Abschnitt L3 mit konstanter Wandstärke von s3 = 3 mm übergeht. Die große Wandstärke ist im unteren Rohrbereich mit hoher Flüssigkeitssäule (Mikroalgensuspension 4) und entsprechend hohem Flüssigkeitsdruck angeordnet. Im mittleren Abschnitt L2 nimmt die Wandstärke 2 kontinuierlich ab und mündet in dem dritten Abschnitt L3, wo eine niedrige Flüssigkeitssäule mit entsprechend geringem Innendruck anliegt. Die Reduzierung der Wandstärke erfolgt dabei einseitig an der Rohrinnenseite, wodurch das Rohr über seine Länge einen konstanten Außendurchmesser aufweist. Das innere Rohr 2 ist im Ausführungsbeispiel mit Wasser 5 gefüllt. Diese Wassersäule dient dem Druckausgleich gegenüber der Flüssigkeitssäule (Mikroalgensuspension 4) und minimiert hierdurch die Belastung des inneren Rohres, welches daher eine konstante Wandstärke aufweisen kann.

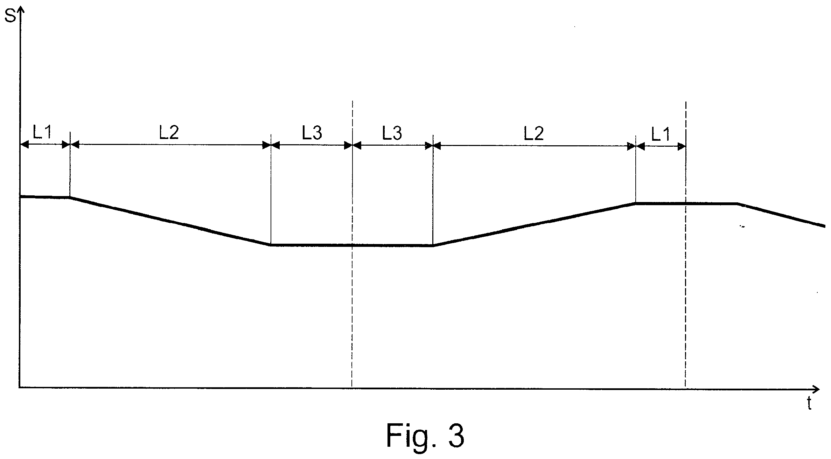

In Figur 3 ist die Wanddicke s eines äußeren Rohres 1 in einem Endlos- Extrudierprozess schematisch über der Zeit t des Extrudiervorgangs dargestellt. Jeweils zwischen zwei benachbarten Abschnitten L3 bzw. zwei benachbarten Abschnitten L1 stoßen zwei Rohre 1 aneinander, welche in einem nachfolgenden Schneidprozess entlang der gestrichelt dargestellten Trennlinie voneinander getrennt werden.

## Patentansprüche

1. Rohrleitung zur senkrecht ausgerichteten Anordnung in einem Photobioreaktor zur Herstellung von Mikroalgenkonzentrat, umfassend zwei konzentrisch angeordnete Rohre unterschiedlichen Durchmessers, zwischen denen ein Kanal für eine Mikroalgensuspension (4) gebildet ist, **dadurch gekennzeichnet, dass** die beiden Rohre (1, 2) aus lichtdurchlässigem Kunststoff hergestellt sind, wobei wenigstens das äußere Rohr (1) über seine Länge wenigstens zwei Abschnitte (L1, L2, L3) mit unterschiedlicher Wandstärke (s) aufweist.

2. Rohrleitung nach Anspruch 1, **dadurch gekennzeichnet, dass** wenigstens ein Abschnitt (L2) eine sich linear verjüngende Wandstärke (s) aufweist.

3. Rohrleistung nach Anspruch 1 oder 2, **dadurch gekennzeichnet, dass** die beiden Rohre (1, 2) über ihre Länge einen konstanten Außendurchmesser aufweisen.

4. Rohrleitung nach einem der vorgenannten Ansprüche, **dadurch gekennzeichnet, dass** wenigstens das äußere Rohr (1), bevorzugt beide Rohre (1, 2) aus Polymethylmethacrylat (PMMA) hergestellt sind.

5. Rohrleitung nach einem der Ansprüche 1 bis 3, **dadurch gekennzeichnet, dass** wenigstens das äußere Rohr (1), bevorzugt beide Rohre (1, 2) aus Polycarbonat hergestellt sind und auf ihrer dem Kanal zugewandten Seite mit einer lebensmittelkonformen Schicht (12, 22) und auf ihrer dem Kanal abgewandten Seite mit einer UV-Sperrschicht (13,23) versehen sind.

6. Rohrleitung nach einem der vorgenannten Ansprüche, **dadurch gekennzeichnet, dass** die beiden Rohre (1, 2) eine Lichtdurchlässigkeit von wenigstens 80 Prozent, vorzugsweise wenigstens 85 Prozent aufweisen.

7. Rohrleitung nach Anspruch 5 oder 6, **dadurch gekennzeichnet, dass** die UV-Sperrschichten (13, 23) derart ausgebildet sind, dass sie für Lichtwellen mit einer Wellenlänge von kleiner 350 nm undurchlässig und mit einer Wellenlänge von 350 nm bis 700 nm durchlässig sind.

8. Rohrleitung nach Anspruch 7, **dadurch gekennzeichnet, dass** die UV-Sperrschichten (13, 23) aus einem Polycarbonatmaterial gebildet sind, dem ein UV-Adsorber zugesetzt ist.

9. Rohrleitung nach einem der Ansprüche 5 bis 8, **dadurch gekennzeichnet, dass** die lebensmittelkonformen Schichten (12, 22) aus Polyethylenterephthalat oder Polycarbonat gebildet sind.

10. Rohrleitung nach einem der Ansprüche 5 bis 9, **dadurch gekennzeichnet, dass** die UV-Sperrschichten (13, 23) und die lebensmittelkonformen Schichten (12, 22) mit dem jeweiligen Rohrgrundkörper (11, 21) homogen und ohne Haftvermittler gebunden sind.

11. Rohrleitung nach einem der vorgenannten Ansprüche, **dadurch gekennzeichnet, dass** Mittel zur Verringerung der Lichtbrechung angeordnet sind.

12. Rohrleitung nach Anspruch 11, **dadurch gekennzeichnet, dass** die Mittel zur Verringerung der Lichtbrechung durch Prismen und/oder Ziehstreifen gebildet sind.

13. Verfahren zur Herstellung von Rohren (1) für Rohrleitungen nach einem der vorgenannten Ansprüche, **dadurch gekennzeichnet, dass** die Rohre (1) in einem Endlos- Extrusionsprozess gefertigt werden, wobei zunächst ein Abschnitt (L1) mit einer ersten Wandstärke (s1) extrudiert wird, welche Wandstärke bei konstantem Rohraußendurchmesser über die Länge eines sich anschließenden zweiten Abschnitts (L2) linear reduziert wird, deren endseitig erreichte Wandstärke (s3) über einen sich anschließenden dritten Abschnitt (L3) konstant gehalten wird, wobei die vorgenannten Abschnitte (L1, L2, L3) nachfolgend in immer wieder umgekehrter Reihenfolge extrudiert werden, wodurch hintereinander jeweils zwei symmetrisch zueinander angeordnete Rohre (1) gebildet sind, die in einem nachfolgenden Arbeitsschritt über einen Schneidprozess voneinander getrennt werden.

14. Verfahren nach Anspruch 13, **dadurch gekennzeichnet, dass** die Rohre aus Polymethylmethacrylat (PMMA) gefertigt werden.

15. Verfahren nach Anspruch 13, **dadurch gekennzeichnet, dass** die Rohre aus Polycarbonat gefertigt werden, wobei eine UV-Sperrschicht (13,23), eine Polycarbonat-Strukturschicht (11, 21) und eine Lebensmittelkontaktschicht- (12, 22) coextrudiert werden, wobei das coextrudierte Dreischichtrohr (1, 2) nachfolgend über temperierte und gestaffelte Kühlstufen abgekühlt wird.

## Claims

1. Tubing for use in a vertically aligned photobioreactor for the manufacture of microalgae concentrate comprising two concentrically arranged tubes of different diameters between which a canal has been formed for a microalgae suspension (4), **characterized in that** both tubes (1, 2) are made of light-permeable plastic, whereby at least the outer tube (1) over its length has at least two sections (L1, L2, L3) with different wall strengths (s).

2. Tubing according to claim 1, **characterized in that** at least one section (L2) has a linear tapering wall thickness (s).

3. Tubing according to claim 1 or 2, **characterized in that** both tubes (1, 2) have a constant outer diameter over their length.

4. Tubing according to one of the aforementioned claims, **characterized in that** at least the outer tube (1), preferably both tubes (1, 2), are made of polymethyl methacrylate (PMMA).

5. Tubing according to one of the aforementioned claims, **characterized in that** at least the outer tube (1), preferably both tubes (1, 2), are made of polycarbonate and are provided with a layer (12, 22) conforming to foodstuffs regulations on the side facing the canal and with an UV blocking layer (13, 23) on the side facing away from the canal.

6. Tubing according to one of the aforementioned claims, **characterized in that** both tubes (1, 2) have a light permeability of at least 80 percent, preferably at least 85 percent.

7. Tubing according to claim 5 or 6, **characterized in that** the UV blocking layers (13, 23) are formed in such a way that they are impermeable for light waves with a wavelength of less than 350 nm and are permeable for wavelengths from 350 nm to 700 nm.

8. Tubing according to claim 7, **characterized in that** the UV blocking layers (13, 23) are made of polycarbonate material to which a UV adsorber has been added.

9. Tubing according to one of the claims 5 to 8, **characterized in that** the layers conforming to foodstuffs regulations (12, 22) are made of polyethylene terephthalate or polycarbonate.

10. tubing according to one of the claims 5 to 9, **characterized in that** the UV blocking layers (13, 23) and the layers conforming to foodstuffs regulations (12, 22) are bonded with the respective tubing base body (11, 21) homogenously and without bonding agent.

11. Tubing according to one of the aforementioned claims, **characterized in that** means for reducing light refraction are attached.

12. tubing according to claim 11, **characterized in that** the means for reducing light refraction are formed by prisms and/or drawlines.

13. Process for the production of tubing (1) for pipelines according to one of the aforementioned claims, **characterized in that** the tubing (1) is manufactured in an endless extrusion process, whereby initially a section (L1) is extruded with the first wall thickness (s1) which, while retaining a constant tubing outer diameter, is reduced linearly over the length of a subsequent second section (L2), whereby the end wall thickness (s3) is maintained constant over a subsequent third section (L3), whereby the aforementioned sections (L1, L2, L3) are thereafter extruded in repeatedly reverse order, as a result of which two tubes (1) arranged symmetrically to each other are formed respectively in succession, which in a following work stage are separated from each other in a cutting process.

14. Process according to claim 13, **characterized in that** the tubing is made of polymethyl methacrylate (PMMA).

15. Process according to claim 13, **characterized in that** the tubing is made of polycarbonate, whereby a UV blocking layer (13, 23), a polycarbonate structure layer (11, 21) and a foodstuffs contact layer (12, 22) are co-extruded, whereby the co-extruded three-layer tubing (1, 2) is cooled thereafter via temperature-controlled and graduated cooling stages.

## Revendications

1. Canalisation destinée à être placée verticalement dans un photobioréacteur pour la fabrication de concentré de micro-algues, comprenant deux tubes concentriques de diamètres différents, entre lesquels est formé un canal pour une suspension de micro-algues (4), **caractérisée en ce que** les deux tubes (1, 2) sont réalisés en plastique laissant passer la lumière, au moins le tube extérieur (1) présentant sur sa longueur au moins deux sections (L1, L2, L3) d'épaisseurs de paroi (s) différentes.

2. Canalisation selon la revendication 1, **caractérisée en ce que** au moins une section (L2) présente une épaisseur de paroi (s) s'amincissant de manière linéaire.

3. Canalisation selon la revendication 1 ou 2, **caractérisée en ce que** les deux tubes (1, 2) présentent sur leur longueur un diamètre extérieur constant.

4. Canalisation selon l'une des revendications ci-dessus, **caractérisée en ce que** au moins le tube extérieur (1), de préférence les deux tubes (1, 2), sont réalisés en polyméthacrylate de méthyle (PMMA).

5. Canalisation selon l'une des revendications 1 à 3, **caractérisée en ce que** au moins le tube extérieur (1), de préférence les deux tubes (1, 2), sont réalisés en polycarbonate et sont munis, sur leur côté tourné vers le canal, d'une couche de qualité alimentaire (12, 22) et, de leur côté tourné du côté opposé au canal, d'une couche barrière bloquant les rayons UV (13, 23).

6. Canalisation selon l'une des revendications ci-dessus, **caractérisée en ce que** les deux tubes (1, 2) présentent une transparence à la lumière d'au moins 80%, de préférence d'au moins 85%.

7. Canalisation selon la revendication 5 ou 6, **caractérisée en ce que** les couches barrières bloquant les rayons UV (13, 23) sont réalisées de telle sorte qu'elles ne puissent être traversées par des ondes lumineuses d'une longueur inférieure à 350 nm et puissent être traversées par des ondes lumineuses d'une longueur de 350 nm à 700 nm.

8. Canalisation selon la revendication 7, **caractérisée en ce que** les couches barrières bloquant les rayons UV (13, 23) sont réalisées dans un polycarbonate auquel est ajouté un adsorbant de rayons UV.

9. Canalisation selon l'une des revendications 5 à 8, **caractérisée en ce que** les couches de qualité alimentaire (12, 22) sont réalisées en polyéthylène téréphtalate ou en polycarbonate.

10. Canalisation selon l'une des revendications 5 à 9, **caractérisée en ce que** les couches barrières bloquant les rayons UV (13, 23) et les couches de qualité alimentaire (12, 22) sont reliées de manière homogène et sans agent de collage au corps de tube respectif (11, 21).

11. Canalisation selon l'une des revendications ci-dessus, **caractérisée en ce que** des moyens de réduction de la réfraction de la lumière sont mis en place.

12. Canalisation selon la revendication 11, **caractérisée en ce que** les moyens de réduction de la réfraction de la lumière sont des prismes et/ou des rubans étirés.

13. Procédé de fabrication de tubes (1) pour des canalisations selon l'une des revendications ci-dessus, **caractérisé en ce que** les tubes (1) sont fabriqués dans un processus d'extrusion sans fin, une section (L1) étant tout d'abord moulée par extrusion avec une première épaisseur de paroi (s1), laquelle épaisseur de paroi est réduite de manière linéaire sur la longueur d'une deuxième section (L2) consécutive, le diamètre extérieur du tube restant constant, l'épaisseur de paroi (s3) obtenue à sa fin étant maintenue constante sur une troisième section (L3) consécutive, lesdites sections (L1, L2, L3) étant moulées ensuite par extrusion dans un ordre toujours inverse, de sorte que sont formés l'un après l'autre respectivement deux tubes (1) placés symétriquement l'un vers l'autre, séparés par un processus de coupe à une étape de travail suivante.

14. Procédé selon la revendication 13, **caractérisé en ce que** les tubes sont réalisés en polyméthacrylate de méthyle (PMMA).

15. Procédé selon la revendication 13, **caractérisé en ce que** les tubes sont réalisés en polycarbonate, une couche barrière bloquant les rayons UV (13, 23), une couche structurée en polycarbonate (11, 21) et une couche de qualité alimentaire (12, 22) étant co-extrudées, le tube à trois couches co-extrudé (1, 2) étant ensuite refroidi par des étapes de refroidissement à température réglée et échelonnées.
